# EUROPEAN PATENT APPLICATION

(11) **EP 2 796 141 A1**
(43) Date of publication of application: **29.10.2014**
(21) Application number: 12860321.4
(22) Date of filing: 21.12.2012
(51) Int. Cl.: A61K 33/06, A61K 9/16, A61P 35/00, A61P 35/04

(54) **CANCER CELL-INHIBITING CERAMICS AND METHOD FOR PRODUCING CANCER CELL-INHIBITING CERAMICS, METHOD FOR TREATING BONE NEOPLASM, AND USE OF B TRICALCIUM PHOSPHATE POROUS GRANULES HAVING GRAIN SIZE OF 1 TO 10 MM**

(30) Priority: 22.12.2011 JP 2011281529
(71) Applicant: Olympus Corporation, Shibuya-ku Tokyo 151-0072 (JP); National University Corporation Tokyo Medical and Dental University, Bunkyo-ku Tokyo 113-8510 (JP)
(72) Inventor: SEKIYA, Ichiro, Tokyo 113-8510 (JP); MUNETA, Takeshi, Tokyo 113-8510 (JP); HAKAMATSUKA, Yasuharu, Tokyo 151-0072 (JP)
(74) Representative: Schicker, Silvia
(86) International application number: PCT/JP2012/083202
(87) International publication number: WO 2013/094722

(57) **Abstract**

Proliferation of cancer cells is effectively inhibited. Provided is a cancer cell-inhibiting ceramic containing a β-tricalcium phosphate porous granule with a particle size of 1 to 10 µm. The β-tricalcium phosphate porous granule is taken up into cancer cells at an affected area and have an effect of inhibiting proliferation of a cancer tissue. The β-tricalcium phosphate porous granule with a particle size of 1 to 10 µm can be used to more effectively inhibit the proliferation of the cancer cells.

## Description

### {Technical Field}

The present invention relates to a cancer cell-inhibiting ceramic, a process for producing the ceramic, a method for treating a bone tumor, and use of a β-tricalcium phosphate porous granule with a particle size of 1 to 10 µm.

### {Background Art}

Conventionally, cancer cell inhibitors that inhibit cancer cell metastasis and recurrence have been known (see, for example, Patent Literature 1). Patent Literature 1 discloses an effect that a film is used to apply a β-tricalcium phosphate porous body, thereby inhibiting cancer cell proliferation, metastasis, and recurrence.

### {Citation List}

### {Patent Literature}

{PTL 1}
Japanese Unexamined Patent Application, Publication No. 2010-126434

### {Summary of Invention}

### {Technical Problem}

PTL 1 discloses a cancer cell inhibitor using a β-tricalcium phosphate porous body with a particle size of 25 to 75 µm. Unfortunately, cancer cells are unlikely to phagocytose the porous body with this particle size.

It is an object of the present invention to provide a cancer cell-inhibiting ceramic capable of effectively inhibiting cancer cell proliferation, a process for producing a cancer cell-inhibiting ceramic, a method for treating a bone tumor, and use of β-tricalcium phosphate porous granules with a particle size of 1 to 10 µm.

### {Solution to Problem}

### In order to achieve the above objective, the present invention provides the following solutions.

The first aspect of the present invention provides a cancer cell-inhibiting ceramic containing a β-tricalcium phosphate porous granule with a particle size of 1 to 10 µm.

The present inventors have conducted research and have found out that use of β-tricalcium phosphate porous granules with a granule size of 1 to 10 µm enables cancer cells to incorporate the granules to inhibit proliferation of the cancer cells. The β-tricalcium phosphate porous granules according to this aspect have a larger surface area and a higher solubility than non-porous granules of β-tricalcium phosphate. Accordingly, the porous granules are likely to affect the cancer cells after their incorporation. According to this aspect, the cancer cell proliferation can be thus effectively inhibited.

In the above aspect, the β-tricalcium phosphate porous granule with a size of 1 to 10 µm may be produced from a β-tricalcium phosphate porous body having a porosity of 20 to 90%.

With such a configuration, β-tricalcium phosphate porous granules are easily dissolved and thus taken up into cancer cells.

The second aspect of the present invention provides a cancer cell-inhibiting ceramic containing a β-tricalcium phosphate porous granule with a particle size of 1 to 10 µm and a β-tricalcium phosphate porous granule with a particle size of 10 to 100 µm.

The study by the inventors has demonstrated that macrophages accumulate around the β-tricalcium phosphate porous granules with a particle size of 10 to 100 µm. The cancer cell-inhibiting ceramic according to this aspect may be disposed on an affected area after a cancer tissue such as a bone tumor has been removed. Even if the cancer tissue does remain in that affected area, the β-tricalcium phosphate porous granules with a particle size of 1 to 10 µm should effectively inhibit proliferation of the tumor. In addition, macrophages are involved in bone regeneration. Accordingly, the β-tricalcium phosphate porous granules with a particle size of 10 to 100 µm allow macrophages to accumulate in that affected area. This should achieve more effective bone regeneration.

In the above aspect, the β-tricalcium phosphate porous granule with a particle size of 1 to 10 µm and the β-tricalcium phosphate porous granules with a particle size of 10 to 100 µm may be produced from a β-tricalcium phosphate porous body having a porosity of 20 to 90%.

With such a configuration, β-tricalcium phosphate porous granules with a size of 1 to 10 µm are easily dissolved and thus taken up into cancer cells. Also, β-tricalcium phosphate porous granules with a size of 10 to 100 µm are easy to be dissolved.

The third aspect of the present invention provides a method for treating a bone tumor, the method including: applying a cancer cell-inhibiting ceramic containing a β-tricalcium phosphate porous granule with a particle size of 1 to 10 µm onto a site and/or a surrounding thereof where a tumor tissue has been removed to treat the tumor.

The fourth aspect of the present invention provides use of a β-tricalcium phosphate porous granule with a particle size of 1 to 10 µm in the manufacture of cancer cell-inhibiting ceramic for the treatment of a bone tumor.

The fifth aspect of the present invention provides a process for producing a β-tricalcium phosphate porous granule, the process including: a synthesis step of synthesizing β-tricalcium phosphate powder by a mechanochemical method; a sintering step of adding a foaming agent to and sintering the β-tricalcium phosphate powder synthesized in the synthesis step to produce a β-tricalcium phosphate porous body; and a classification step of milling the β-tricalcium phosphate porous body produced in the sintering step and classifying a β-tricalcium phosphate porous granule with a particle size of 1 to 10 µm by using an air classification method.

According to this aspect, the classification step is used to classify and produce β-tricalcium phosphate porous granules with a particle size of 1 to 10 µm from β-tricalcium phosphate porous bodies produced in the sintering step, whose particles are in a relatively large block shape. Accordingly, as a series of a process for producing β-tricalcium phosphate porous bodies used depending on their application, β-tricalcium phosphate porous granules with a particle size of 1 to 10 µm may be produced.

### {Advantageous Effects of Invention}

The present invention exerts effects to be able to effectively inhibit cancer cell proliferation.

### {Brief Description of Drawings}

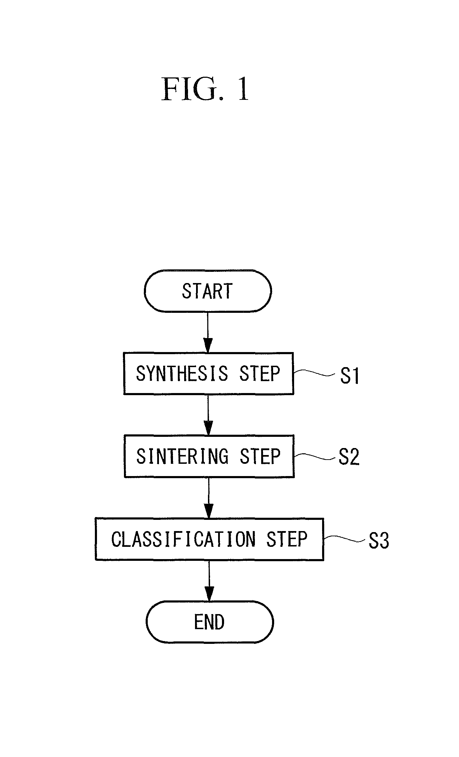

{Fig. 1}
   Fig. 1 is a flow chart illustrating a process for producing β-tricalcium phosphate porous granules according to the first embodiment of the present invention.
{Fig. 2}
   Fig. 2 is a graph showing a particle size distribution of β-tricalcium phosphate porous granules with a particle size of 1 to 10 µm, the granules being produced by the production process shown in Fig. 1.
{Fig. 3}
   Fig. 3 is a photomicrograph obtained when only HT1080 cells were cultured for 1 day.
{Fig. 4}
   Fig. 4 is a photomicrograph obtained when HT1080 cells were cultured for 1 day and then, β-TCP granules were added to a dish.
{Fig. 5}
   Fig. 5 is a photomicrograph obtained when only HT1080 cells were cultured for 5 days.
{Fig. 6}
   Fig. 6 is a photomicrograph obtained when HT1080 cells were cultured for 5 days after addition of β-TCP granules.
{Fig. 7}
   Fig. 7 is a scanning electron microscopic image of an HT1080 cell cultured 3 days after the addition of β-TCP granules.
{Fig. 8}
   Fig. 8 is a light microscopic image obtained when only HT1080 cells were cultured for 3 days.
{Fig. 9}
   Fig. 9 is a light microscopic image obtained when HT1080 cells were cultured for 3 days after addition of 1 mg/ml of β-TCP granules.
{Fig. 10}
   Fig. 10 is a light microscopic image obtained when HT1080 cells were cultured for 3 days after addition of 3 mg/ml of β-TCP granules.
{Fig. 11}
   Fig. 11 is a graph showing particle size distributions of β-tricalcium phosphate porous granules with a particle size of 1 to 10 µm and β-tricalcium phosphate porous bodies with a particle size of 10 to 100 µm, the granules and the bodies being produced by the production process shown in Fig. 1.
{Fig. 12}
   Fig. 12 is a diagram illustrating how to culture SaOS cells by adding β-TCP granules A.
{Fig. 13}
   Fig. 13 is a photomicrograph showing morphology of SaOS cells before addition of β-TCP granules A.
{Fig. 14}
   Fig. 14 is photomicrographs showing living and dead SaOS cells at each additive amount of β-TCP granules A.
{Fig. 15}
   Fig. 15 is a graph showing the results with the Kruskal-Wallis test.
{Fig. 16}
   Fig. 16 is a graph showing the results of an MTT assay.
{Fig. 17}
   Fig. 17 is a picture indicating that macrophages accumulated around β-tricalcium phosphate porous granules with a particle size of 10 to 100 µm and the β-tricalcium phosphate porous granules then disintegrated, which resulted in macrophage activation.

### {Description of Embodiments}

### [First Embodiment]

### <Cancer Cell-inhibiting Ceramic>

With reference to the drawings, the following describes cancer cell-inhibiting ceramic according to the first embodiment of the present invention and a process for producing the ceramic.

The cancer cell-inhibiting ceramic according to this embodiment is composed of β-tricalcium phosphate porous granules with a particle size of 1 to 10 µm.

### <Production Process>

As shown in the flow chart of Fig. 1, a process for producing the cancer cell-inhibiting ceramic according to this embodiment includes: a synthesis step S1 of synthesizing β-tricalcium phosphate powder by a mechanochemical method; a sintering step S2 of adding a foaming agent to and sintering the β-tricalcium phosphate powder synthesized in the synthesis step S1 to produce block-shaped β-tricalcium phosphate porous bodies; and a classification step S3 of milling the β-tricalcium phosphate porous bodies produced in the sintering step S2 and classify β-tricalcium phosphate porous granules with a particle size of 1 to 10 µm by using an air classification method.

The mechanochemical method in the synthesis step S1 is described as follows: a rotating mill, for example, a zirconia ball mill is used to mix materials for β-tricalcium phosphate; and collision energy between substances is utilized to synthesize β-tricalcium phosphate powder.

Specifically, first, calcium hydrogen phosphate dihydrate (CaHPO₄·2H₂O) with a purity of 99.9% and calcium carbonate (CaCO₃) with a purity of 99.99% are mixed at an atomic ratio Ca/P of 1.50. Next, pure water is added thereto to prepare slurry having a solid content of 10% by weight. Then, this slurry is placed in a zirconia ball mill to make a reaction while subjected to grinding for 24 hours. After that, the solid is recovered and is put into a stainless bat. Finally, the solid is dried in an electric drying oven at 80°C.

In the sintering step S2, first, the dried β-tricalcium phosphate solid is subjected to milling, followed by calcination in an electric furnace at 750°C for 1 hour. Next, to the calcinated powder (e.g., 60 g) are added a foaming agent and a deflocculant including ammonium polyacrylate and ethylene nonylphenyl ether (e.g., 1 to 6 ml), and the mixture is subjected to mixing and foaming to prepare slurry. Then, the slurry is injected into a mold. For example, the deflocculant used is Celuna D-305 manufactured by Chukyo Yushi Co., Ltd.

After the slurry is dried at 80°C for 24 hours while being kept in the mold, the temperature is raised at a rate of 100°C per hour and the slurry is sintered at 1050°C for 30 minutes. This process produces β-tricalcium phosphate porous bodies having a porosity of from 20 to 90% (preferably 75%). With regard to the porosity of the β-tricalcium phosphate porous bodies according to this embodiment, a conventional method was used to calculate apparent porosity.

For example, in the classification step S3, a TC-15N manufactured by Nisshin Engineering Inc. was used to classify β-tricalcium phosphate porous bodies with a size of 100 µm or less at a rotating speed of 2500 rpm, a wind level of 2.0 m³/min, and a feed rate of 1.0 kg/h. Then, β-tricalcium phosphate porous granules with a size of 10 µm or less were recovered.

### <Particle Size Distribution Measurement>

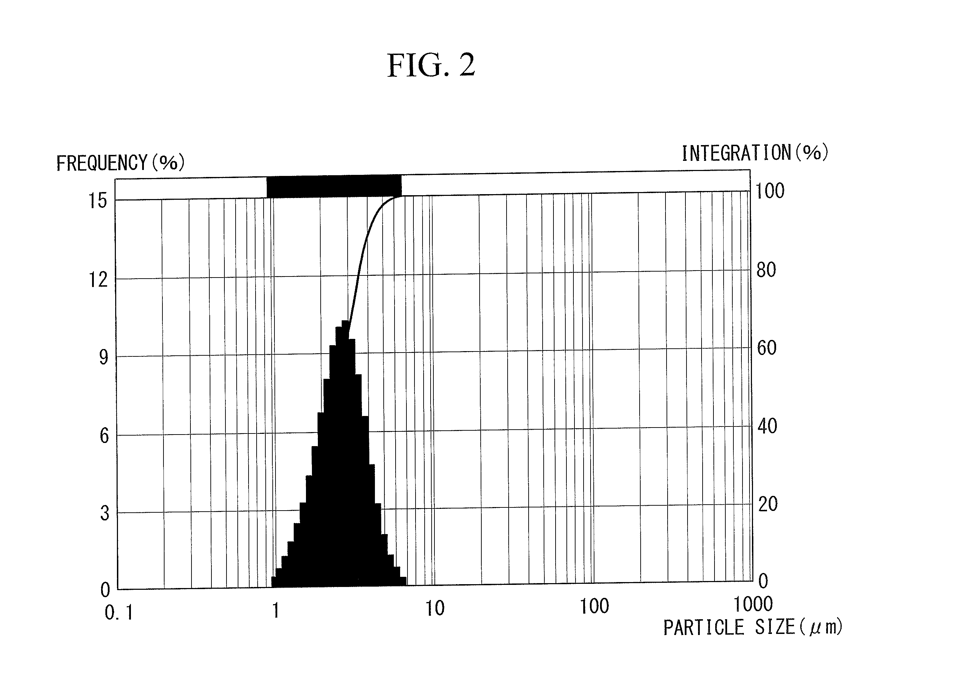

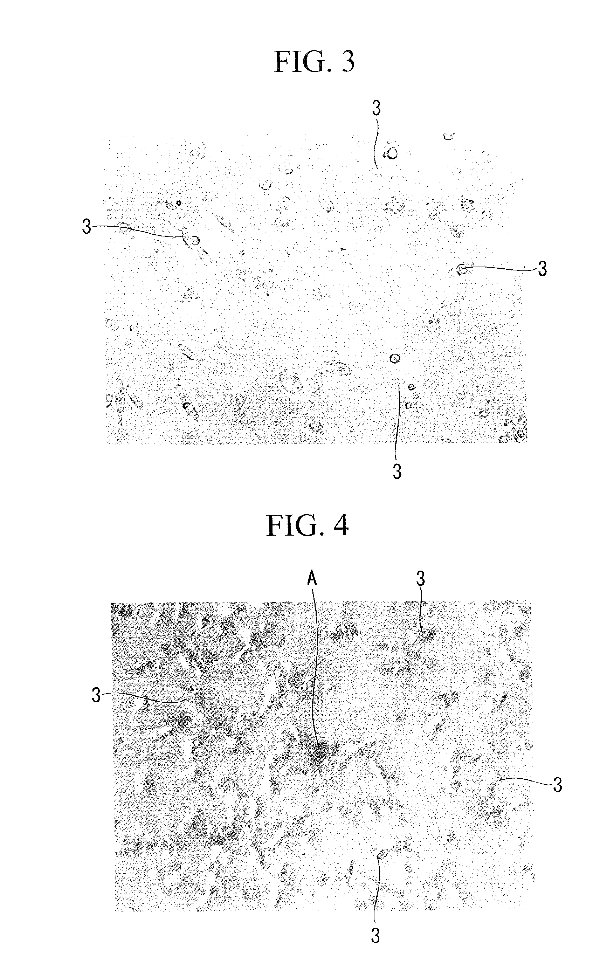

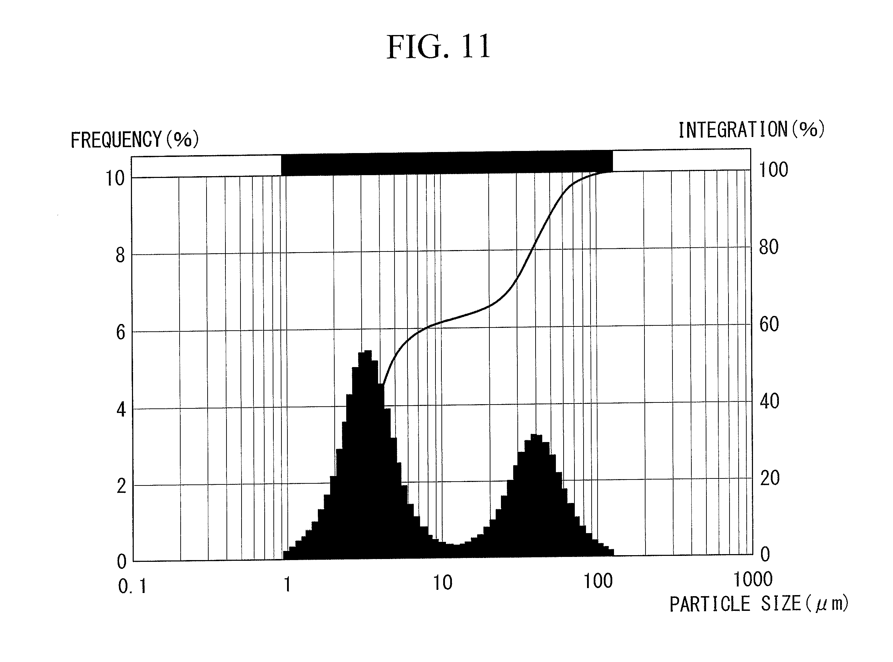

A particle size distribution of β-tricalcium phosphate porous granules with a particle size of 1 to 10 µm (i.e., cancer cell-inhibiting ceramic; hereinafter, simply referred to as "β-TCP granules A"; see Fig. 4) as produced using a production process according to this embodiment, was determined. As shown in Fig. 2, the results demonstrated that the distribution, for example, had a peak at a particle size of 3 µm. In Fig. 2, the ordinate indicates a frequency (%) and integration (%). The abscissa indicates a particle size (µm). The same applies to Fig. 11.

A particle size distribution of β-tricalcium phosphate porous granules is determined as follows: for example, distilled water is used as a dispersion medium and Triton X-100 is used as a dispersant; dispersion is carried out using an ultrasonic bath (150 W) for 1 minute; and a Microtrac HRA is then used to determine the distribution by a laser diffraction/scattering method.

The following describes effects of cancer cell-inhibiting ceramic as so prepared and a process for producing the ceramic.

When the cancer cell-inhibiting ceramic according to this embodiment is attached to cancer cells 3 (see, for example, Fig. 3), the cancer cells take up β-tricalcium phosphate porous granules with a particle size of 1 to 10 µm. This inhibits proliferation of the cancer cells.

Differing from an anti-cancer drug such as cisplatin, β-tricalcium phosphate porous granules are composed of only materials having a high bioaffinity. When these β-tricalcium phosphate porous granules are administered to patients, their cancer cells should be inhibited without any adverse effect. Also, the advantages include that a burden on the patients should be reduced. In addition, the β-tricalcium phosphate porous granules produced are a kind of β-tricalcium phosphate porous bodies. Hence, compared with non-porous granules of β-tricalcium phosphate, they have a large surface area and an increased solubility. These characteristics are likely to affect cancer cells after incorporation of the granules.

### <Verification Experiment>

The following describes an inhibitory effect of β-TCP granules A (cancer cell-inhibiting ceramic) on cancer cells.

As shown in Fig. 3, HT1080 cells (human fibrosarcoma cells; cancer cells) 3 were seeded at 5 k/cm² (300 k/dish) on a 10-cm dish (Nunc) and cultured (at 37°C and 5% CO₂). Fig. 3 is a photomicrograph (x 200) obtained when only HT1080 cells 3 were cultured for 1 day.

At the next day, β-TCP granules A were mixed in a medium at a concentration of 3.0 mg/ml. These granules A were added at 10 ml/dish (30 mg/dish) to a dish containing HT1080 cells 3 as shown in Fig. 4. The medium used is, for example, MEM (GIBCO)/FBS (GIBCO) 10%/P.S.A. Fig. 4 is a photomicrograph (x 200) obtained when HT1080 cells 3 were cultured for 1 day and then, β-TCP granules A were added to a dish. The β-TCP granules A were found to accumulate in the cytoplasm.

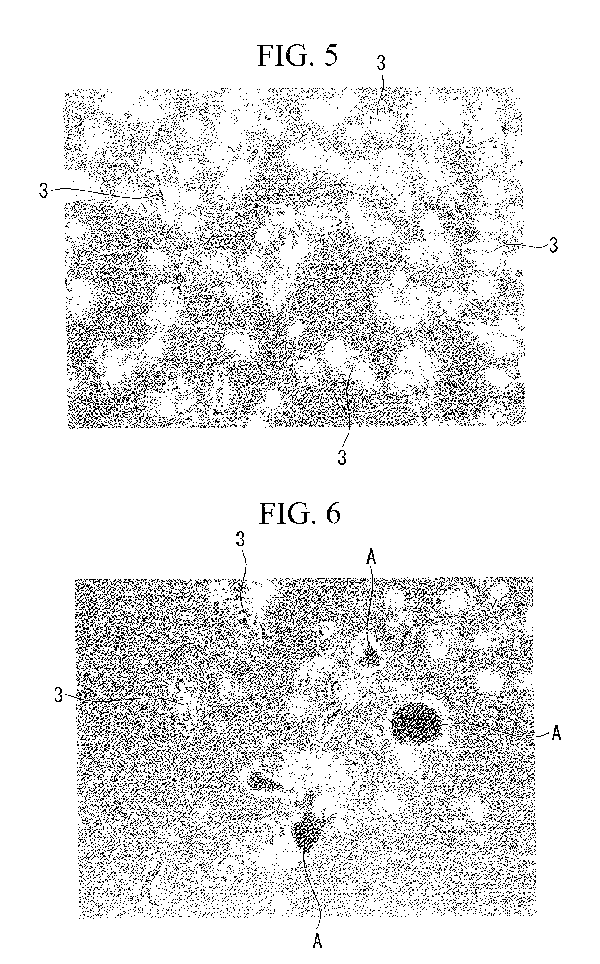

At day 3 of the culture, the β-TCP granules A exhibited a dose dependency and caused the proliferation capability of the HT1080 cells 3 to decrease. Then, the culture was continued. After that, the HT1080 cells 3 containing the β-TCP granules A as shown in Fig. 6 had a more inhibited cell proliferation capability than the HT1080 cells 3 cultured alone as shown in Fig. 5. Figs. 5 and 6 each show a photomicrograph (x 200) at day 5 of the culture.

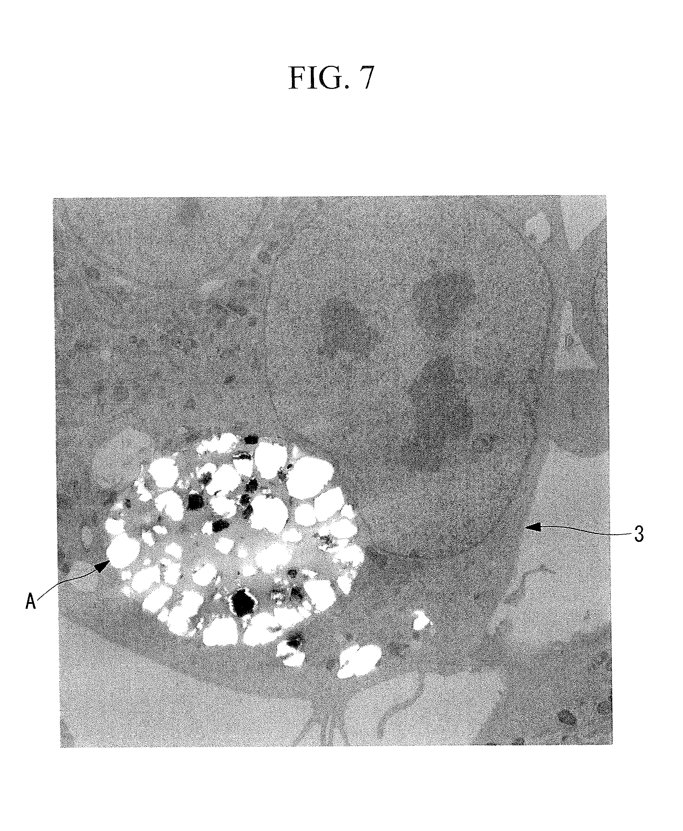

Fig. 7 is a scanning electron microscopic image of an HT1080 cell 3 cultured 3 days after the addition of β-TCP granules A. This image demonstrates that the HT1080 cell 3 took up the β-TCP granules A. Further, HT1080 cells 3 during the culture were observed under a light microscope. The results indicated an inhibited cell proliferation and a contracted cell morphology. Hence, the above demonstrates that incorporating β-TCP granules A causes the proliferation capability of HT1080 cells 3 to be reduced.

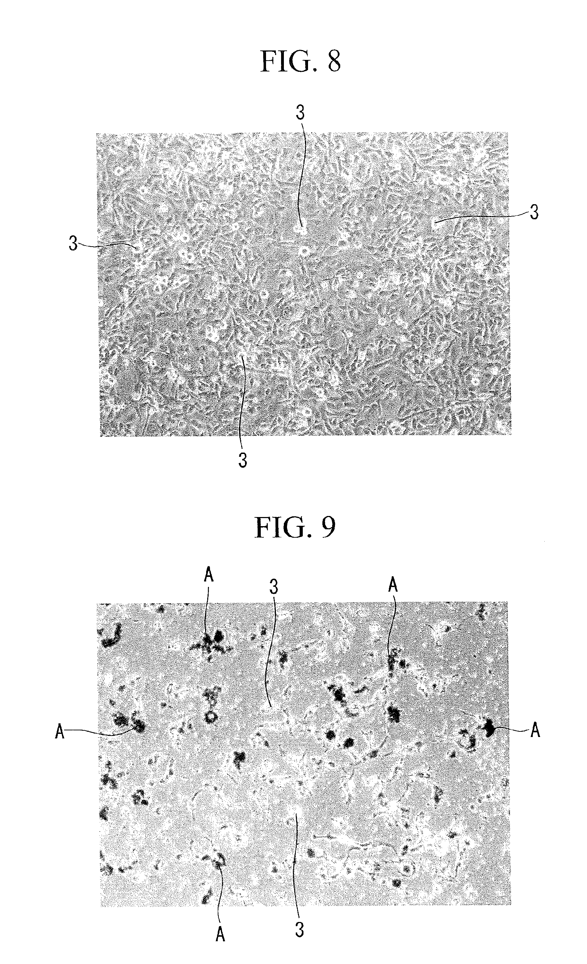

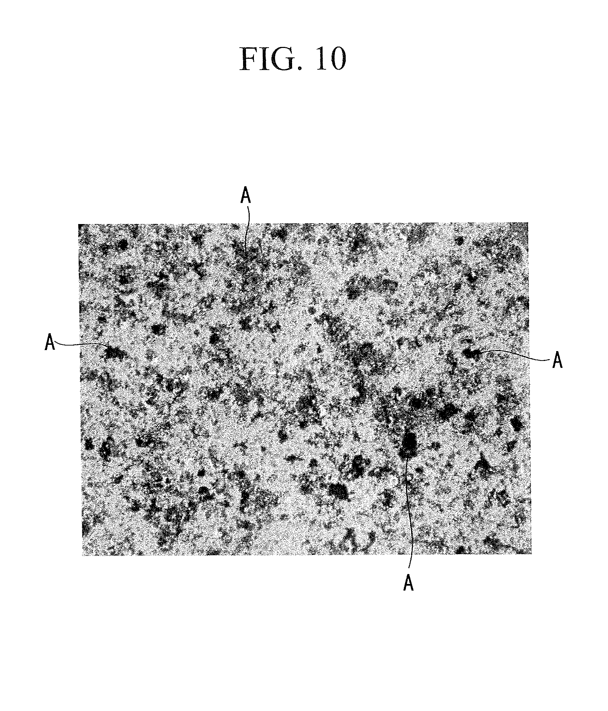

Next, HT1080 cells 3 were cultured on dishes, and β-TCP granules A were added to each dish at 1 mg/ml or 3 mg/ml. These cells were cultured for 3 days. Figs. 8 to 10 demonstrate that as the additive amount of the β-TCP granules A increased, their inhibitory effect increased. Fig. 8 is a light microscopic image obtained when only HT1080 cells 3 were cultured for 3 days. Fig. 9 is a light microscopic image obtained when HT1080 cells were cultured for 3 days after addition of 1 mg/ml of β-TCP granules A. Fig. 10 is a light microscopic image obtained when HT1080 cells were cultured for 3 days after addition of 3 mg/ml of β-TCP granules A.

As described above, the cancer cell-inhibiting ceramic according to this embodiment can effectively inhibit proliferation of cancer cells, because of being composed of β-tricalcium phosphate porous granules with a particle size of 1 to 10 µm (β-TCP granules A). Also, with such a configuration, the β-tricalcium phosphate porous granules, being porous, are easily dissolved and thus taken up into cancer cells. A production process according to this embodiment makes it possible to easily produce such β-tricalcium phosphate porous granules with a particle size of 1 to 10 µm.

### [Second Embodiment

Hereinafter, the elements shared with those of the cancer cell-inhibiting ceramic according to the first embodiment have the same reference signs to avoid description redundancy.

### <Cancer Cell-inhibiting Ceramic>

The following describes cancer cell-inhibiting ceramic according to the second embodiment of the present invention.

The cancer cell-inhibiting ceramic according to this embodiment is composed of β-tricalcium phosphate porous granules with a particle size of 1 to 10 µm (β-TCP granules A) and β-tricalcium phosphate porous granules with a particle size of 10 to 100 µm.

### <Production Process>

For example, in the classification step S3, a TC-15N manufactured by Nisshin Engineering Inc. was used to classify β-tricalcium phosphate porous bodies with a size of 100 µm or less at a rotating speed of 4000 rpm, a wind level of 2.0 m³/min, and a feed rate of 1.0 kg/h. Then, a fraction containing β-tricalcium phosphate with a size of 10 to 100 µm was recovered. Next, the fraction containing β-TCP with a size of 10 to 100 µm was classified at a rotating speed of 2500 rpm, a wind level of 2.0 m³/min, and a feed rate of 1.0 kg/h to recover β-tricalcium phosphate with a size of 10 µm or less.

### <Particle Size Distribution Measurement>

A particle size distribution of β-tricalcium phosphate porous granules with a particle size of 1 to 100 µm (i.e., cancer cell-inhibiting ceramic; hereinafter, simply referred to as "β-TCP granules B" (not shown)) as produced using a production process according to this embodiment was determined. As shown in Fig. 11, for example, the results demonstrated that the distribution had a peak at a particle size of 3 µm within a range from 1 to 10 µm and another peak at a particle size of 40 µm within a range from 10 to 100 µm. The method for determining a particle size distribution of the β-TCP granules B is substantially the same as the method for determining a particle size of the β-TCP granules A according to the first embodiment.

### <Verification Experiment 1>

A verification experiment for the β-TCP granules B is substantially the same as that for the β-TCP granules A according to the first embodiment. First, HT1080 cells 3 were cultured on dishes, and β-TCP granules B were added to each dish at 1 mg/ml or 3 mg/ml. These cells were cultured for 3 days. The β-TCP granules B also exhibited a cancer cell inhibitory effect. However, the β-TCP granules A had a higher cancer cell inhibitory effect than the β-TCP granules B.

### <Verification Experiment 2>

Next, the following describes an inhibitory effect of the β-TCP granules A with a particle size of 1 to 10 µm on human osteosarcoma cells.

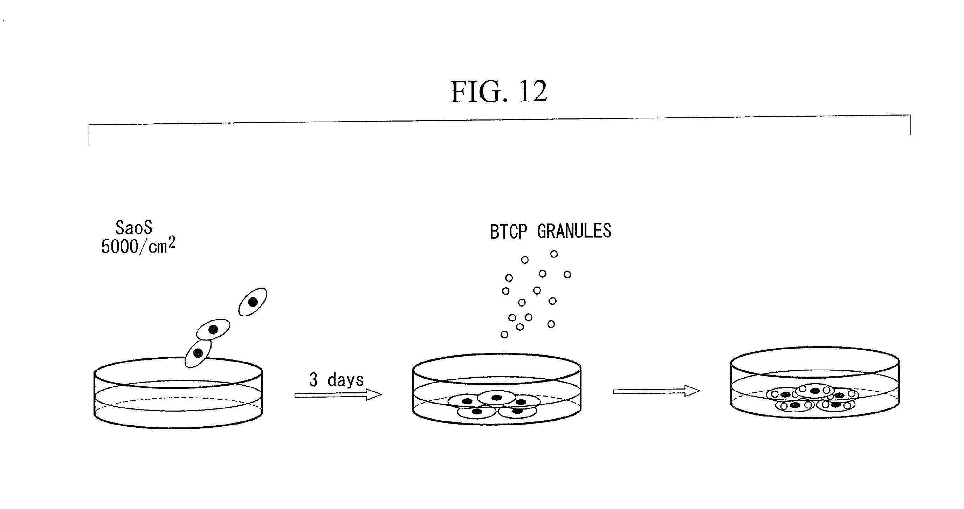

As shown in Fig. 12, human osteosarcoma cells (SaOS-2; hereinafter, referred to as "SaOS cells") 5 were seeded at a density of 5 x 10² (cells/cm²) on each dish and cultured. The SaOS cells 5 were cultured for 3 days on dishes. Then, no β-TCP granules A, 1 mg/ ml, 3 mg/ml, or 5 mg/ml of the β-TCP granules A were added to each dish. The medium was changed twice a week.

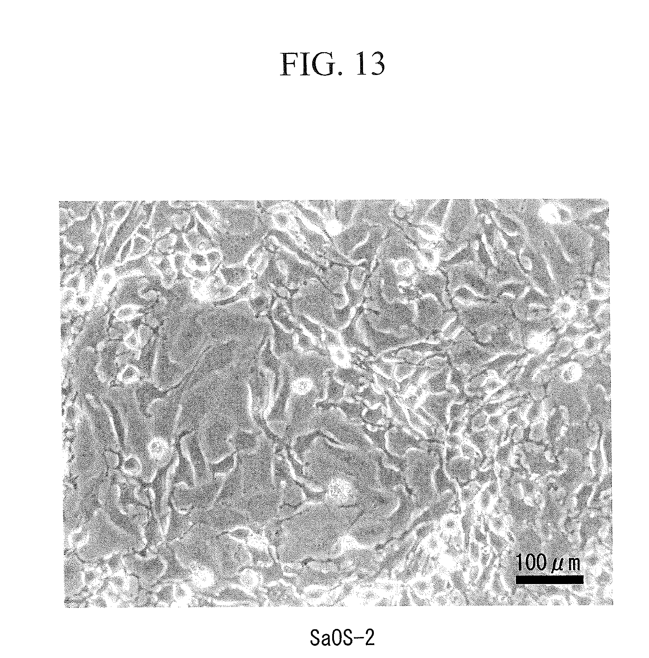

Fig. 13 is a photomicrograph showing morphology of SaOS cells 5 before addition of β-TCP granules A.

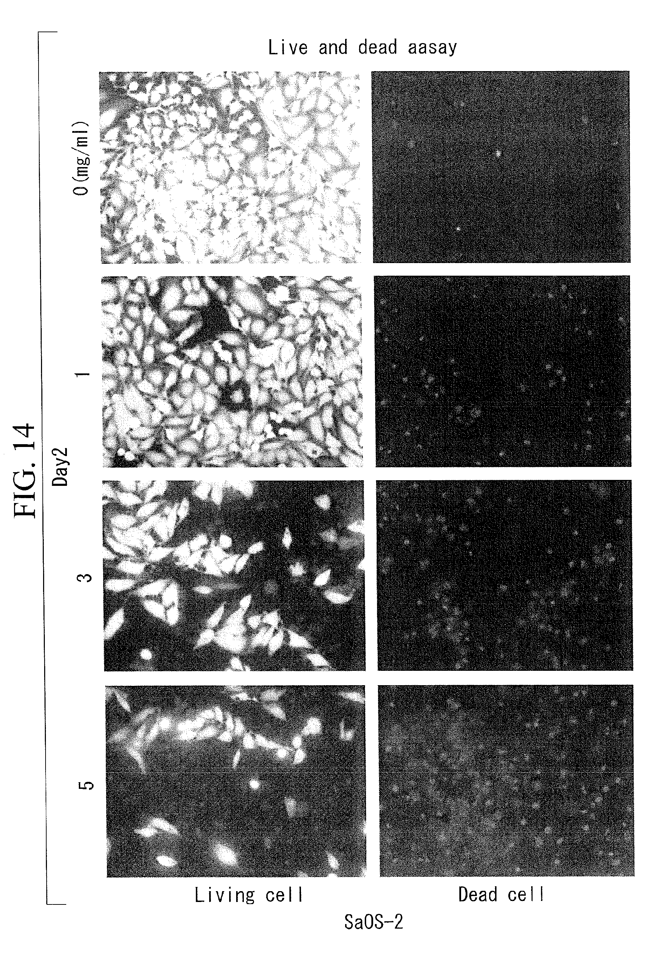

The β-TCP granules A were added to SaOS cells 5, and the cells were further cultured for 2 days. As shown in Fig. 14, the results demonstrated that depending on an amount of the β-TCP granules A, the number of living cells decreased and the number of dead cells increased. Fig. 14 includes: photomicrographs showing living cells of the respective SaOS cells 5 when no β-TCP granules A (0 mg/ml), 1 mg/ ml, 3 mg/ml, or 5 mg/ml of the β-TCP granules A were added; and photomicrographs showing dead cells of the SaOS cells 5.

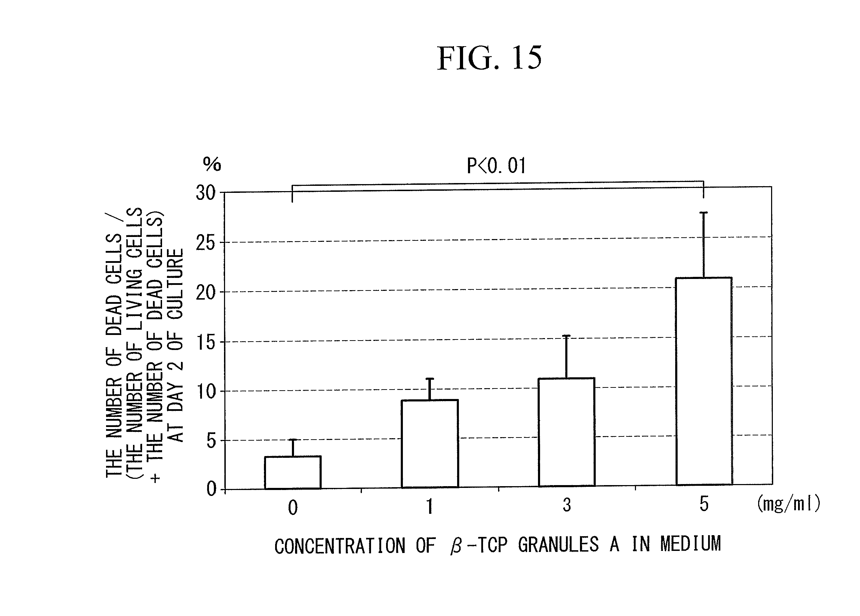

As shown in Fig. 15, a significant difference (P < 0.01, the Kruskal-Wallis test) is found between two groups: one with no β-TCP granules A (0 mg/ml) added and one with 5 mg/ml of the β-TCP granules A added. In Fig. 15, the ordinate represents "The number of dead cells / (The number of living cells + The number of dead cells)" at day 2 of culture. The abscissa represents a concentration (mg/ ml) of β-TCP granules A in a medium.

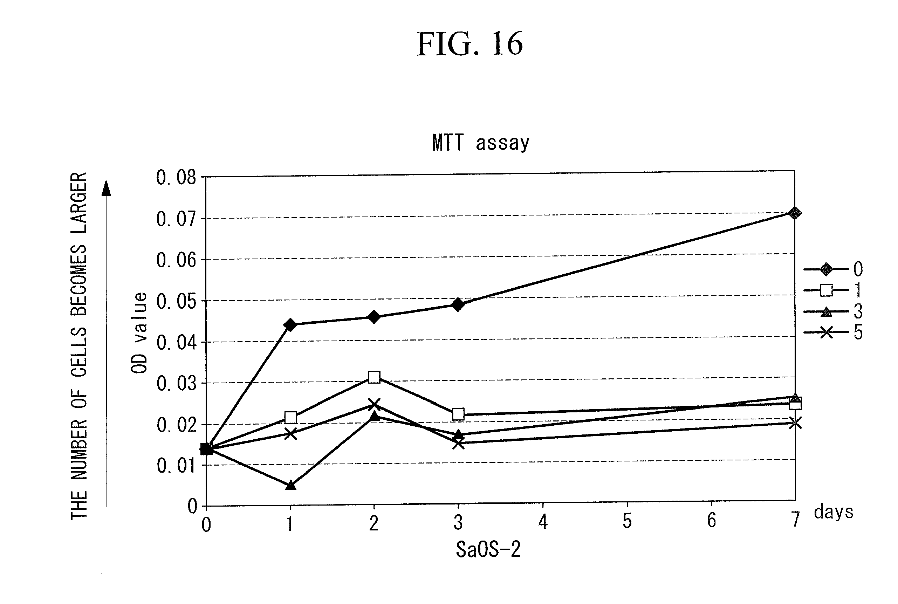

Further, as shown in Fig. 16, an MTT assay showed an inhibition of cell proliferation of β-TCP granules A application groups. In addition, the groups with 3 mg/ml or 5 mg/ml of β-TCP granules A applied tended to show a stronger proliferation inhibitory effect in an earlier period than the group with 1 mg/ml of β-TCP granules A applied. In Fig. 16, the ordinate represents "The number of living cells / (The number of living cells + The number of dead cells)". The abscissa represents days.

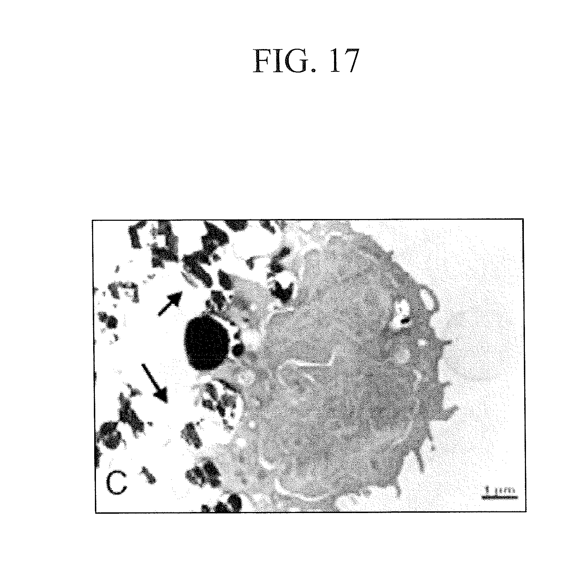

Macrophages may accumulate around β-tricalcium phosphate porous granules with a particle size of 10 to 100 µm. Fig. 17 indicates that macrophages accumulated around β-tricalcium phosphate porous granules with a particle size of about 10 µm and the β-tricalcium phosphate porous granules then disintegrated, which resulted in macrophage activation.

A semicircular object which occupies the center region of Fig. 17 is a macrophage. This macrophage has pod-like protrusions in its surrounding. Thus, the macrophage seems to be activated. In addition, in this figure, the arrows indicate disintegrated β-tricalcium phosphate porous granules.

When β-tricalcium phosphate porous granules with a particle size of 10 to 100 µm are applied on an affected area, macrophages should cause T cells, an immune cell, to accumulate around cancer cells, thereby inhibiting proliferation, metastasis, and recurrence of the cancer cells. The cancer cell-inhibiting ceramic according to this embodiment may be disposed on an affected area after a cancer tissue such as a bone tumor has been removed. In this case, even if the cancer tissue does remain in the affected area, the tumor proliferation should be effectively inhibited. As macrophages are involved in bone regeneration, the β-tricalcium phosphate porous granules with a particle size of 10 to 100 µm should gather macrophages to that affected area and achieve more effective bone regeneration.

In actual use of the cancer cell-inhibiting ceramic in clinical practice, it is considered that the cancer cell-inhibiting ceramic according to the present invention may be applied on a site or its surrounding where a tumor tissue has been removed. This should inhibit cancer cell metastasis and recurrence, and further effectively regenerate bone.

Also, it is considered that the cancer cell-inhibiting ceramic may be mixed with, for example, autologous blood, a Ringer's solution, or a saline, and may fill a site where a tumor tissue has been removed. In order to sufficiently recover a bone mass after the filling, an absorbable artificial bone, an autologous bone, or a mixture thereof may be used to fill the site.

### {Reference Signs List}

- A: β-TCP granule (Cancer cell-inhibiting ceramic)
- 3: HT1080 cell (Cancer cell)
- S1: Synthesis step
- S2: Sintering step
- S3: Classification step

## Claims

1. A cancer cell-inhibiting ceramic comprising a β-tricalcium phosphate porous granule with a particle size of 1 to 10 µm.

2. The cancer cell-inhibiting ceramic according to Claim 1, wherein the β-tricalcium phosphate porous granule is produced from a β-tricalcium phosphate porous body having a porosity of 20 to 90%.

3. A cancer cell-inhibiting ceramic comprising:
a β-tricalcium phosphate porous granule with a particle size of 1 to 10 µm; and
a β-tricalcium phosphate porous granule with a particle size of 10 to 100 µm.

4. The cancer cell-inhibiting ceramic according to Claim 3, wherein the β-tricalcium phosphate porous granule with a particle size of 1 to 10 µm and the β-tricalcium phosphate porous granule with a particle size of 10 to 100 µm are produced from a β-tricalcium phosphate porous body having a porosity of 20 to 90%.

5. A method for treating a bone tumor, the method comprising: applying a cancer cell-inhibiting ceramic comprising a β-tricalcium phosphate porous granule with a particle size of 1 to 10 µm onto a site and/or a surrounding thereof where a tumor tissue has been removed to treat the tumor.

6. Use of a β-tricalcium phosphate porous granule with a particle size of 1 to 10 µm in the manufacture of a cancer cell-inhibiting ceramic for the treatment of a bone tumor.

7. A process for producing a cancer cell-inhibiting ceramic, the process comprising:
a synthesis step of synthesizing β-tricalcium phosphate powder by a mechanochemical method;
a sintering step of adding a foaming agent to and sintering the β-tricalcium phosphate powder synthesized in the synthesis step to produce a β-tricalcium phosphate porous body; and
a classification step of milling the β-tricalcium phosphate porous body produced in the sintering step and classifying a β-tricalcium phosphate porous granule with a particle size of 1 to 10 µm by using an air classification method.
